# EUROPEAN PATENT SPECIFICATION

(11) **EP 4 183 439 B1**
(45) Date of publication and mention of the grant of the patent: **26.08.2026**
(21) Application number: 21843013.0
(22) Date of filing: 14.07.2021
(51) Int. Cl.: A61M 25/00, A61B 17/12, A61M 39/10, A61B 17/00

(54) **MEDICAL INSTRUMENT SET, DELIVERY SYSTEM, AND EMBOLUS DELIVERY MEDICAL SYSTEM**
MEDIZINISCHES INSTRUMENTENSET, ABGABESYSTEM UND EMBOLUSABGABE-MEDIZINSYSTEM
ENSEMBLE D'INSTRUMENTS MÉDICAUX, SYSTÈME D'ADMINISTRATION ET SYSTÈME MÉDICAL D'ADMINISTRATION D'EMBOLE

(30) Priority: 16.07.2020 JP 2020122112
(43) Date of publication of application: 24.05.2023
(73) Proprietor: Terumo Kabushiki Kaisha, Tokyo 151-0072 (JP)
(72) Inventor: SHIBATA, Hideaki, Ashigarakami-gun, Kanagawa 259-0151 (JP); IKUNO, Eri, Ashigarakami-gun, Kanagawa 259-0151 (JP)
(74) Representative: Casalonga
(86) International application number: PCT/JP2021/026419
(87) International publication number: WO 2022/014632

(56) References cited:
- WO-A1-2017/035365
- JP-A- 2013 103 073
- JP-A- 2020 089 501
- JP-A- S6 242 049
- US-A1- 2007 142 859
- US-A1- 2008 103 477
- US-B1- 6 746 426

## Description

### Technical Field

The present invention relates to a delivery system according to the preamble of independent claim 1, such as it is e.g. known from WO2017/035365 A1.

### Background Art

There is no drug treatment to prevent an aneurysm (aortic aneurysm) generated in an aorta of a patient from increasing in diameter and rupture, and surgical treatment (surgery) is generally performed for the aneurysm in diameter with a risk of rupture. Further, in a surgery of the aortic aneurysm of the related art, artificial blood vessel replacement surgery in which an artificial blood vessel is transplanted by laparotomy or thoracotomy has been a mainstream, and in recent years, application of lower invasive stent graft interpolation (endovascular aneurysm repair: EVAR) has been rapidly expanding.

For example, in stent graft interpolation for an abdominal aortic aneurysm (AAA), a catheter having a stent graft at a dista1 end thereof is inserted from a peripheral blood vessel of the patient, and the stent graft is expanded and indwells in an aneurysm lesion, thereby blocking a blood flow to the aneurysm and preventing the aneurysm from rupturing.

In general, a stent graft used in the stent graft interpolation has a structure in which two types of members, that is, a "main body portion" including bifurcated portions bifurcated into a substantially Y-shape and "leg portions" attached to the bifurcated portions and attached to a right iliac artery and a left iliac artery, respectively, are assembled.

Therefore, in the stent graft interpolation, so-called "endoleak" in which the blood flow remains in the aneurysm may occur due to blood leakage from a periphery of the stent graft due to insufficient adhesion of the interpolated stent graft, backflow of blood from a thin blood vessel (side branch blood vessel) branched from the aneurysm, or the like. In this case, since pressure is applied to a wall of the aneurysm by the blood flow entering the aneurysm, there is a potential risk of rupture of the aneurysm.

The following PTL 1 discloses a device including a catheter capable of holding a compressed relatively elongated sponge (embolization material) in a lumen thereof and a plunger for pushing out the embolization material held in the catheter into an aneurysm filled with blood in order to block blood flow remaining in an aortic aneurysm due to endoleak. Since the sponge used in this device expands immediately when exposed to the blood, the sponge is pushed out into the aneurysm and expands when absorbing the blood in the aneurysm, and the sponge indwells in the aneurysm in this state to block the blood flow and prevent rupture.

### Citation List

### Patent Literature

PTL 1: US Patent No. 9561096

### Summary of Invention

### Technical Problem

The invention is defined in independent claim 1. Certain optional features of the invention are defined in the dependent claims.

In endoleak embolization including the technique disclosed in PTL 1, the following "direct insertion method" and "indirect insertion method" are conceivable as embolization material delivery methods for delivering an embolization material into an aneurysm through a catheter.

The "direct insertion method" is a delivery method including: a procedure for inserting a first catheter in which an embolization material is loaded into a sheath of a second catheter indwelling in a body lumen (procedure A1); a procedure for inserting a delivery pusher into the first catheter while a distal end of the first catheter reaches the aneurysm to deliver the embolization material into the aneurysm (procedure A2); and a procedure for removing the first catheter and the delivery pusher from the second catheter after the embolization material indwells in the aneurysm (procedure A3). In the direct insertion method, the embolization material is pushed out into the aneurysm and indwells therein by inserting and pushing out the delivery pusher into the first catheter.

The "indirect insertion method" is a delivery method including: a procedure for inserting part of the distal end of the first catheter loaded with the embolization material into the sheath of the second catheter indwelling in the body lumen (procedure Bl); a procedure for inserting a loading pusher into the first catheter to transfer the embolization material to the second catheter (procedure B2); a procedure for removing the loading pusher and the first catheter from the second catheter (procedure B3); a procedure for inserting the delivery pusher into the second catheter to deliver the embolization material into the aneurysm (procedure B4); and a procedure for removing the delivery pusher from the second catheter after the embolization material indwells in the aneurysm (procedure B5). In the indirect insertion method, the embolization material is moved from the first catheter to the second catheter by the loading pusher, and is pushed out into the aneurysm and indwelling therein by inserting and pushing out the delivery pusher into the second catheter.

However, these two delivery methods have the following problems (problems 1 to 3), and it can be said that there is room for improvement, particularly in terms of the complexity of the procedures and procedure time.

### (Problem 1)

In the direct insertion method, the first catheter requires flexibility and kink resistance such that the first catheter can be inserted following a shape of the meandering blood vessel. In order to fulfill these functions, it is necessary to make a flexible catheter body, and there is a risk that the loaded embolization material may be damaged during packaging or unpacking. When the embolization material indwells in the aneurysm, both the first catheter and the delivery pusher should be inserted into the aneurysm along a meandering flow path of the blood vessel, which can increase the difficulty of the procedures.

### (Problem 2)

In the indirect insertion method, since the number of procedures is larger than that in the direct insertion method, and procedures such as insertion and removal of a device are complicated, the procedure time may be longer.

### (Problem 3)

Both the direct insertion method and the indirect insertion method are methods for pushing out the embolization material into the aneurysm in a state where at least a part of the first catheter to which the embolization material is loaded is inserted into the second catheter. Accordingly, an outer diameter of the first catheter is smaller than an inner diameter of the second catheter, and an outer diameter of the embolization material is smaller than an inner diameter of the first catheter. Therefore, in order to make a required amount of small-diameter embolization material indwell in the aneurysm, the number of inserted embolization material inevitably increases, and the procedure time may be long.

At least one embodiment of the invention has been made in view of the above circumstances, and specifically, aims at providing a delivery system comprising a medical instrument set for delivering an embolization material into an aneurysm, with said system being capable of shortening procedure time by simplifying a procedure while reducing the number of procedure steps.

### Solution to Problem

In order to solve this problem, the present invention provides a delivers system according to independent claim 1. The dependent claims relate to advantageous embodiments.

### Advantageous Effect of Invention

According to at least one embodiment of the invention, it is possible to shorten the procedure time by simplifying the procedure while reducing the number of procedures.

### Brief Description of Drawings

[FIG. 1] FIG. 1 is a diagram showing configurations of a medical instrument set and a delivery set according to the present embodiment.
[FIG. 2] FIG. 2 is a diagram showing a configuration of an embolization material delivery medical system according to the present embodiment.
[FIG. 3A] FIG. 3A is a schematic cross-sectional view showing one configuration example of an engagement portion of the medical instrument set.
[FIG. 3B] FIG. 3B is a schematic cross-sectional view showing an engaged state of the engagement portion in FIG. 3A.
[FIG. 4A] FIG. 4A is a schematic cross-sectional view showing another configuration example of the engagement portion of the medical instrument set.
[FIG. 4B] FIG. 4B is a schematic cross-sectional view showing an engaged state of the engagement portion in FIG. 4A.
[FIG. 5] FIG. 5 is a schematic cross-sectional view showing a monuted state between an embolization material loading catheter and a delivery catheter.
[FIG. 6A] FIG. 6A is a partially enlarged view of a vicinity of a handle portion of a delivery pusher.
[FIG. 6B] FIG. 6B is a partially enlarged view showing an inserted state of the delivery pusher.
[FIG. 7A] FIG. 7A is an operation example of the embolization material delivery medical system according to the present embodiment, showing a state where the delivery catheter is delivered into an aneurysm.
[FIG. 7B] FIG. 7B is an operation example of the embolization material delivery medical system according to the present embodiment, showing a state where a stent graft is deployed in the aneurysm.
[FIG. 7C] FIG. 7C is an operation example of the embolization material delivery medical system according to the present embodiment, showing a state before the embolization material loading catheter is mounted to the delivery catheter.
[FIG. 7D] FIG. 7D is an operation example of the embolization material delivery medical system according to the present embodiment, showing a state where the embolization material loading catheter is mounted to the delivery catheter.
[FIG. 7E] FIG. 7E is an operation example of the embolization material delivery medical system according to the present embodiment, showing a state where the delivery pusher is being inserted into the embolization material loading catheter.
[FIG. 7F] FIG. 7F is an operation example of the embolization material delivery medical system according to the present embodiment, showing a state where the embolization material is pushed out into the aneurysm by the delivery pusher.
[FIG. 7G] FIG. 7G is an operation example of the embolization material delivery medical system according to the present embodiment, showing a state where the embolization material loading catheter is detached from the delivery catheter.
[FIG. 8A] FIG. 8A is a modification of the embolization material loading catheter according to the present embodiment, showing a state before the engagement portion is engaged.
[FIG. 8B] FIG. 8B is a modification of the embolization material loading catheter according to the present embodiment, showing a state after the engagement portion is engaged.

### Description of Embodiments

Hereinafter, an embodiment of the invention will be described in detail with reference to the drawings. Here, an embodiment to be described later is a specific example of a technical idea of the invention, and the invention is not limited thereto.

Further, in the drawings attached to the present specification, for convenience of illustration and understanding, a scale, an aspect ratio, a shape, and the like may be changed from actual ones and may be schematically expressed as appropriate, and the drawings are just examples and do not limit the interpretation of the invention.

In the present specification, in an operation direction of each part constituting an embolization material delivery medical system 300, for example, a side on which an embolization material 10 is to be conveyed into an aneurysm in a direction along an axial direction of a delivery catheter 40 is referred to as a "distal side (or a distal portion)", and a side that is located on a side opposite to the distal side in the axial direction and on which a surgeon performs an operation with hands (a side from which the delivery catheter 40 is removed) is referred to as a "proximal side (or a proximal portion)". In the present specification, a "distal end" means a certain range in the axial direction including a most distal end, and a "proximal end" means a certain range in the axial direction including a most proximal end.

A medical instrument set 100, a delivery system 200, and the embolization material delivery medical system 300 according to the present embodiment may be applied to endoleak embolization for stent graft interpolation of an abdominal aortic aneurysm (AAA), which is an example of a treatment method for preventing rupture of an aneurysm generated in a blood vessel. In addition, the treatment method in which the medical instrument set 100, the delivery system 200, and the embolization material delivery medical system 300 according to the present embodiment are used is not limited to the endoleak embolization, and is also applicable to other intervention treatment methods for preventing the rupture of the aneurysm generated in the blood vessel.

### [Configuration]

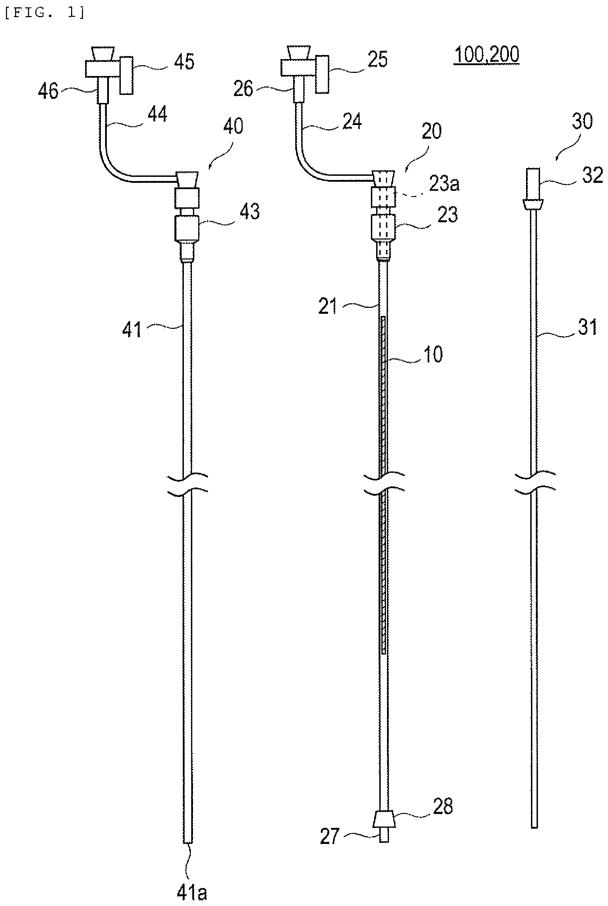

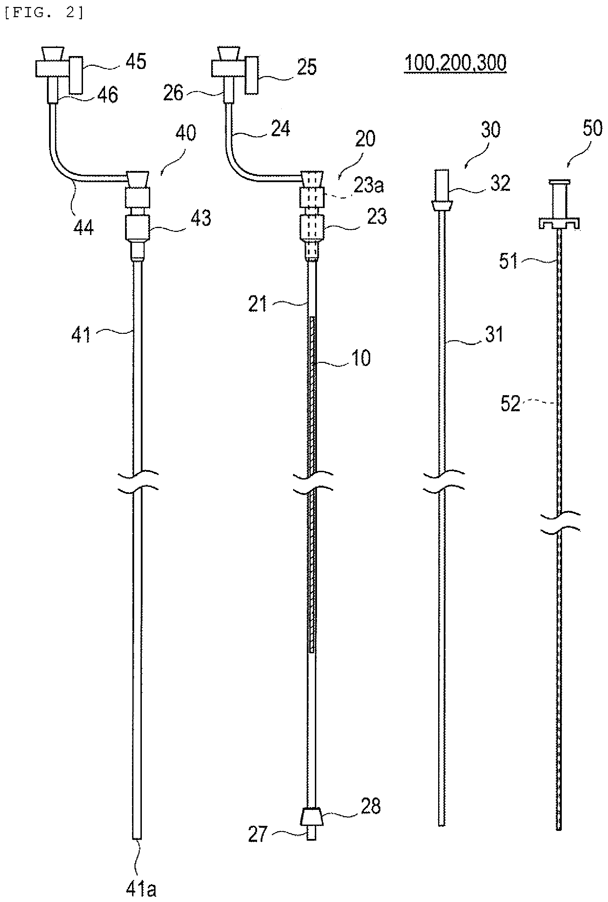

Next, configurations of the medical instrument set 100, the delivery system 200, and the embolization material delivery medical system 300 according to the present embodiment will be described. FIG. 1 shows each device constituting the medical instrument set 100 and the delivery system 200 according to the present embodiment, and FIG. 2 shows each device constituting the embolization material delivery medical system 300 according to the present embodiment.

First, the embolization material 10 used in the medical instrument set 100, the delivery system 200, and the embolization material delivery medical system 300 according to the present embodiment will be described.

### (Embolization material)

The embolization material 10 indwells in an aneurysm such as an aortic aneurysm generated in the blood vessel and expands by absorbing a liquid containing blood flowing into the aneurysm. The embolization material 10 is loaded into an embolization material loading catheter 20, and with the embolization material loading catheter 20 mounted to the delivery catheter 40, the embolization material 10 is pushed out by a delivery pusher 30 and indwells in the aneurysm.

The embolization material 10 is an elongated fibrous linear body (umbilical member) made of an expandable material (polymer material (water-absorbing gel material) or the like) that expands by contacting with an aqueous liquid containing the blood under a physiological condition. The embolization material 10 is an elongated umbilical member having a substantially circular cross-sectional shape in a direction orthogonal to a longitudinal direction, and is relatively fragile before expansion in which the embolization material 10 indwells in the aneurysm. The cross-sectional shape of the embolization material 10 is not particularly limited, and may be a polygonal shape in addition to the substantially circular shape.

Here, the "physiological condition" means a condition having at least one environmental characteristic in a body or on a body surface of a mammal (for example, humans). Such a characteristic includes an isotonic environment, a pH buffering environment, an aqueous environment, a pH near neutral (about 7), or a combination thereof. The "aqueous liquid" includes, for example, body fluids of a mammal (for example, humans) such as isotonic fluid, water, blood, spina1 fluid, plasma, serum, glass body fluid, and urine. An outer diameter of the embolization material 10 may be accommodated within inner diameters of the embolization material loading catheter 20 and the delivery catheter 40, and may be substantially equal to the inner diameters of these catheters. A total length of the embolization material 10 is not particularly limited, and can be appropriately set depending on factors such as a size of the aneurysm to indwell in consideration of ease of loading and shortening of procedure time.

A constituent material for the embolization material 10 is not particularly limited as long as the constituent material is a material that expands by absorbing at least a liquid such as blood, and has no (or extremely low) harmful effect on a human body even in a state of indwelling in the aneurysm. In addition, the embolization material 10 may be added with a visualization material whose existing position in a living body can be confirmed by a confirmation method such as an X-ray, a fluorescent X-ray, an ultrasound, a fluorescence method, an infrared ray, or an ultraviolet ray.

### <Medical Instrument Set>

Next, the configuration of the medical instrument set 100 according to the present embodiment will be described. As shown in FIG. 1, the medical instrument set 100 according to the present embodiment includes the embolization material loading catheter 20 and the delivery pusher 30.

### (Embolization material Loading Catheter)

The embolization material loading catheter 20 includes an elongated catheter body 21 in which a loading lumen 22 is provided, a proximal hub 23 provided on a proximal side of the catheter body 21, and a flexible tube 24 having one end connected to a proximal side of the proximal hub 23 and the other end connected to a port 26 of a three-way stopcock 25.

The catheter body 21 is a tubular member formed with a hole (the loading lumen 22) penetrating from an opening portion on a distal side to an opening portion on the proximal side along an axial direction. A length of the catheter body 21 in an extending direction is appropriately defined, and it is sufficient that the catheter body 21 has a length at least capable of accommodating the embolization material 10.

An inner diameter of the loading lumen 22 is designed to be substantially equal to an inner diameter of a sheath lumen 42 of the delivery catheter 40. Accordingly, the outer diameter of the embolization material 10 is substantially equal to inner diameters of the embolization material loading catheter 20 and the delivery catheter 40, and it is not necessary to reduce a diameter size of the embolization material 10 as in a direct insertion method and an indirect insertion method which can be assumed as a delivery method for the embolization material 10. Therefore, when a required amount of the embolization material 10 indwells in the aneurysm, the number of inserted embolization material 10 can be reduced, and procedure time can be shortened.

The embolization material loading catheter 20 is mainly provided in a state where the embolization material 10 is loaded in advance, and the embolization material 10 to be loaded into the catheter body 21 may be loaded into the catheter body 21 by the surgeon or the like grasping the embolization material 10. As a method of loading the embolization material 10, the surgeon can grip the embolization material 10 and insert the embolization material 10 from a distal connection portion 27 side or a proximal hub 23 side of the embolization material loading catheter 20.

The catheter body 21 is mounted by being engaged with a sheath hub 43 of the delivery catheter 40 in a state where the embolization material 10 is accommodated by an engagement portion 60, which will be described later. In this mounted state, the delivery pusher 30 is inserted from the proximal hub 23 to push the loaded embolization material 10 toward the delivery catheter 40.

Since the embolization material loading catheter 20 is not used by being inserted into a sheath of the delivery catheter 40 as in the direct insertion method, the embolization material loading catheter 20 does not require flexibility to follow a bent shape of a body lumen during insertion. Therefore, a constituent material for the catheter body 21 is not particularly limited as long as the constituent material is a material that is at least more rigid than the delivery catheter 40 and provides an appropriate degree of hardness to prevent breakage of the loaded embolization material 10 during packaging or the like. As examples of the constituent material for the catheter body 21, a resin material such as a polymer material including polyolefins (for example, polyethylene, polypropylene, polybutene, an ethylene-propylene copolymer, an ethylene-vinyl acetate copolymer, an ionomer, or a mixture of two or more types thereof), a polyolefin elastomer, a crosslinked polyolefin, polyvinyl chloride, polyamide, a polyamide elastomer, polyester, polyester elastomer, polyurethane, polyurethane elastomer, a fluorous resin, polycarbonate, polystyrene, polyacetal, polyimide, polyetherimide, and aromatic polyether ketone, or a mixture thereof, and a metal material such as a shape memory alloy, stainless steel, tantalum, titanium, platinum, gold, or tungsten can be suitably used.

Since it is sufficient that the catheter body 21 is more rigid than a sheath 41 from the viewpoint of preventing breakage of the embolization material 10, in addition to making the material for the catheter body 21 rigid, the catheter body 21 may be configured such that the catheter body 21 is thickened so as not to be kinked when the same material as that of the sheath 41 is used. When the thickness is variable, an outer diameter of the catheter main body 21 is larger than an outer diameter of the sheath 41, but since the embolization material loading catheter 20 is mounted to the delivery catheter 40 via the engagement portion 60, there is no particular problem.

The proximal hub 23 is an intermediate member that includes an insertion passage 23a (lumen) that allows communication between the loading lumen 22 of the catheter body 21 and the tube 24, and allows a fluid (such as physiological saline for priming) flowing from the three-way stopcock 25 to flow to the catheter body 21 via the tube 24. The embolization material 10 loaded into the loading lumen 22 is pushed toward the delivery catheter 40 by the delivery pusher 30 being inserted into the loading lumen 22 via the insertion passage 23a of the proximal hub 23.

A constituent material for the proximal hub 23 is not particularly limited as long as the constituent material is a hard material such as a hard resin. As examples of the constituent material for the proximal hub 23, polyolefins such as polyethylene or polypropylene, polyamide, polycarbonate, or polystyrene can be suitably used.

A hemostasis valve (not shown) is attached into the proximal side of the proximal hub 23. As the hemostasis valve, for example, a substantially elliptical film-shaped (disk-shaped) valve body made of silicone rubber, latex rubber, butyl rubber, or isoprene rubber which is an elastic member may be used.

The one end of the tube 24 is interlocked to the proximal side of the proximal hub 23, and the other end is interlocked to the port 26 of the three-way stopcock 25. The tube 24 is a conduit through which a liquid such as physiological salt solution flowing out from a priming syringe (not shown) interlocked to the port 26 flows.

A constituent material for the tube 24 is not particularly limited as long as the constituent material is a flexible resin material in consideration of operability. As the constituent material for the tube 24, polyolefins such as polyethylene, polypropylene, and ethylene-propylene copolymer, polyester such as polyethylene terephthalate, polystyrene, or polyvinyl chloride can be suitably used.

The three-way stopcock 25 communicates with the loading lumen 22 of the catheter body 21 via the insertion passage 23a of the proximal hub 23 and the tube 24. In addition to a proximal side of the tube 24, a priming syringe for priming the loading lumen 22 of the catheter body 21 can be connected to the port 26 of the three-way stopcock 25.

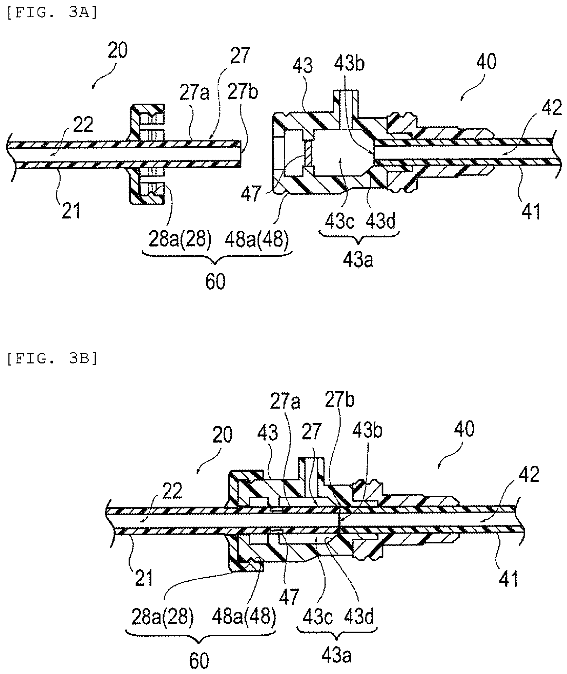

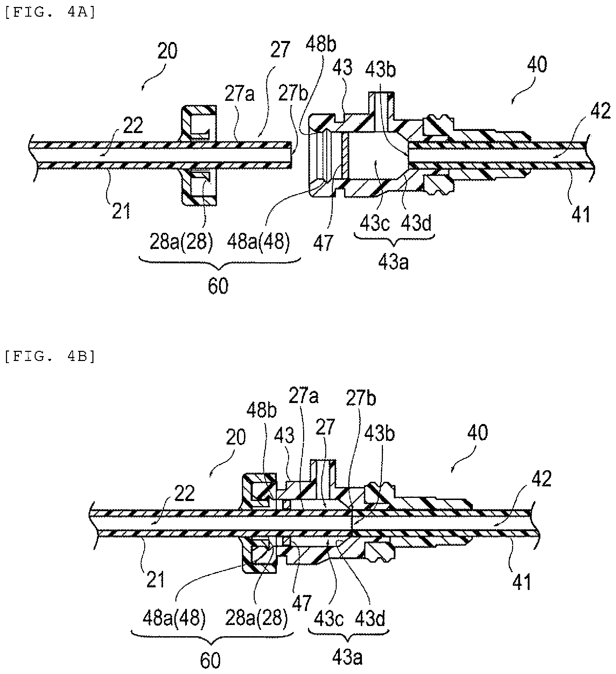

As shown in FIGS. 3 and 4, a distal side of the embolization material loading catheter 20 is provided with the distal connection portion 27 that is connected to the sheath hub 43 of the delivery catheter 40. The distal connection portion 27 is provided with a second engagement portion 28 that engages with a first engagement portion 48 provided on a proximal side of the sheath hub 43. In the medical instrument set 100 according to the present embodiment, the first engagement portion 48 and the second engagement portion 28 constitute the engagement portion 60 for maintaining a connected state between the embolization material loading catheter 20 and the delivery catheter 40.

FIGS. 3 and 4 show configuration examples of the engagement portion 60. In the engagement portion 60 of each configuration, the first engagement portion 48 functions as a female portion, and the second engagement portion 28 functions as a male portion.

As shown in FIG. 3A, the engagement portion 60 may be configured such that the embolization material loading catheter 20 is fitted outside the delivery catheter 40. In this configuration, the first engagement portion 48 is provided as a groove portion 48a provided along a circumferential direction on an outer circumferential surface of the sheath hub 43 on the proximal side. The second engagement portion 28 is provided as a separate skirt member fixed on an outer circumferential surface of a distal portion of the catheter body 21, and includes a plurality of engagement claws 28a provided so as to cover at least a part of the outer circumference of the distal portion of the catheter body 21. As shown in FIG. 3B, the engagement portion 60 maintains the connected state between the embolization material loading catheter 20 and the delivery catheter 40 by the engagement claws 28a of the second engagement portion 28 fitted into the groove portion 48a, which is the first engagement portion 48. When the embolization material loading catheter 20 and the delivery catheter 40 are in the connected state, the loading lumen 22 and the sheath lumen 42 communicate with each other.

As shown in FIG. 4A, the engagement portion 60 may be configured such that the embolization material loading catheter 20 is fitted inside the delivery catheter 40. In this configuration, the first engagement portion 48 is provided as a groove portion 48a formed in an inner wall surface of an inserting portion 48b, which is a passage provided in a substantially central portion of an end surface of the sheath hub 43 on the proximal side. The second engagement portion 28 is provided as a separate skirt member fixed on the outer circumferential surface of the distal portion of the catheter body 21, and includes a plurality of engagement claws 28a disposed so as to be able to fit into the groove portion 48a when the second engagement portion 28 protrudes in a distal direction and is inserted into the inserting portion 48b. As shown in FIG. 4B, the engagement portion 60 maintains the connected state between the embolization material loading catheter 20 and the delivery catheter 40 by the engagement claws 28a fitted into the groove portion 48a. When the embolization material loading catheter 20 and the delivery catheter 40 are in the connected state, the loading lumen 22 and the sheath lumen 42 communicate with each other.

A configuration of the engagement portion 60 is not limited to fitting forms shown in FIGS. 3 and 4 as long as the connected state between the embolization material loading catheter 20 and the delivery catheter 40 is maintained, and can also employ other connection configurations, such as a threaded type. The engagement portion 60 is configured to maintain a mounted state between the embolization material loading catheter 20 and the delivery catheter 40, and prevents the mounted state of the embolization material loading catheter 20 and the delivery catheter 40 from being detached during the procedure. However, the embolization material loading catheter 20 and the delivery catheter 40 do not necessarily have to be engaged via the engagement portion 60. For example, a state where the distal connection portion 27 is inserted into the sheath hub 43 may be the mounted state between the embolization material loading catheter 20 and the delivery catheter 40.

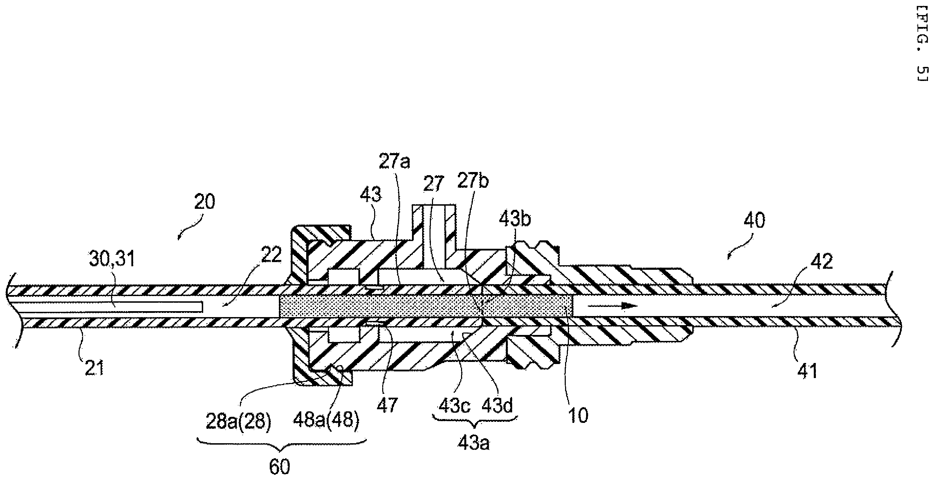

As shown in FIG. 5, the inner diameter of the loading lumen 22 is designed to be substantially equal to the inner diameter of the sheath lumen 42, and accordingly, the outer diameter of the embolization material 10 is also designed, and the embolization material 10 can have a diameter size larger than that of the direct insertion method or the indirect insertion method. Accordingly, in the endoleak embolization, the number of inserted embolization material 10 can be reduced, and as a result, the procedure time can be shortened. Since the inner diameter of the loading lumen 22 is substantially equal to the inner diameter of the sheath lumen 42, in the connected state between the embolization material loading catheter 20 and the delivery catheter 40 caused by the engagement portion 60, a step (clearance) between an opening portion of a distal contact portion 27b and an opening portion on a proximal side of a communication distal portion 43b can be made close to zero. Therefore, when a pusher body 31 of the delivery pusher 30 is inserted into the sheath lumen 42 from the loading lumen 22, the embolization material 10 can be smoothly moved from the embolization material loading catheter 20 to the delivery catheter 40.

The distal connection portion 27 includes an insertion portion 27a that is inserted into a communicating enlarged-diameter portion 43c provided inside the sheath hub 43 when the embolization material loading catheter 20 and the delivery catheter 40 are in the connected state. In the connected state between the embolization material loading catheter 20 and the delivery catheter 40, the insertion portion 27a is inserted into the sheath hub 43 such that the loading lumen 22 and the sheath lumen 42 are aligned in the axial direction. Accordingly, the embolization material 10 is pushed out to the sheath lumen 42 without being exposed to an outside from the loading lumen 22 via the sheath hub 43.

In the connected state between the embolization material loading catheter 20 and the delivery catheter 40, the insertion portion 27a includes the distal contact portion 27b that comes into contact with an inner surface of a tapered portion 43d provided in the communicating enlarged-diameter portion 43c on a distal side thereof. When the insertion portion 27a is inserted into the communicating enlarged-diameter portion 43c, the distal contact portion 27b comes into contact with the tapered portion 43d, so that the loading lumen 22 and the sheath lumen 42 communicate with each other so as not to intersect with other spaces including a space of the communicating enlarged-diameter portion 43c. Therefore, when the embolization material 10 is pushed out from the embolization material loading catheter 20 into the delivery catheter 40, the embolization material 10 is prevented from being damaged (bent or crushed on a distal side) due to abutment of the embolization material 10 against an inner wall surface of the sheath hub 43, is prevented from being erroneously inserted into other spaces within the sheath hub 43 (for example, erroneously entering into a tube 44 connected to the sheath hub 43), and is reliably sent out to the sheath lumen 42.

### (Delivery Pusher)

The delivery pusher 30 is an elongated rod-shaped member inserted into the proximal hub 23 and configured to push out the embolization material 10 accommodated in the catheter body 21 and deliver the embolization material 10 into the aneurysm via the sheath lumen 42 of the delivery catheter 40. The delivery pusher 30 includes the rod-shaped pusher body 31 and a handle portion 32 provided on a proximal side of the pusher body 31 and held by the surgeon when the embolization material 10 is to be delivered into the aneurysm.

In a state where the embolization material loading catheter 20 is mounted to the delivery catheter 40, when the surgeon performs a predetermined operation while gripping the handle portion 32, the delivery pusher 30 pushes the embolization material 10 loaded in the loading lumen 22 into the aneurysm via the sheath lumen 42 of the delivery catheter 40. Specifically, the delivery pusher 30 is pushed out along axial directions of the embolization material loading catheter 20 and the delivery catheter 40 to push out the embolization material 10 loaded in the embolization material loading catheter 20 to an outside (into the aneurysm).

In the mounted state where the embolization material loading catheter 20 is mounted to the delivery catheter 40, a body length of the pusher body 31 of the delivery pusher 30 is longer than a distance from a proximal end of the insertion passage 23a of the proximal hub 23 to the distal end opening 41a of the sheath 41 of the delivery catheter 40 (an opening portion on a distal side communicating with the sheath lumen 42). Therefore, in the state where the embolization material loading catheter 20 is mounted to the delivery catheter 40, when the delivery pusher 30 is inserted from the proximal hub 23, the embolization material 10 loaded in the loading lumen 22 can be passed through the insertion passage 23a→ the sheath hub 43→ the sheath lumen 42 in this order and pushed out into the aneurysm by a single push-out operation.

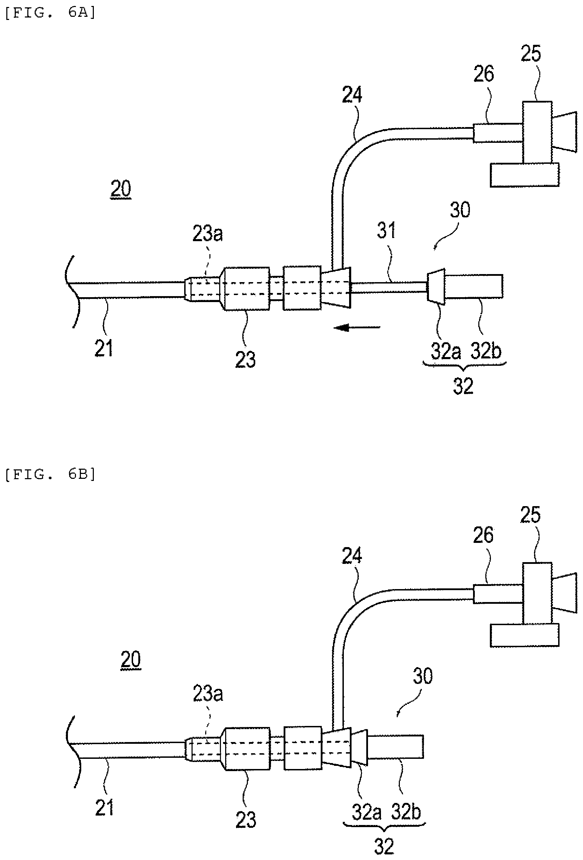

As shown in FIG. 6A, the handle portion 32 has a substantially mushroom shape having a large-diameter head portion 32a on a distal side and a small-diameter handle portion 32b extending to a proximal side of the large-diameter head portion 32a, and an outer diameter dimension of the large-diameter head portion 32a, which is a maximum outer diameter of the handle portion 32, is designed to be larger than an inner diameter dimension of the insertion passage 23a of the proximal hub 23. Accordingly, as shown in FIG. 6B, when the delivery pusher 30 is inserted into the embolization material loading catheter 20, since the large-diameter head portion 32a is not inserted into the insertion passage 23a of the proximal hub 23, an insertion length of the delivery pusher 30 can be limited. Since the handle portion 32 does not enter the proximal hub 23 due to the large-diameter head portion 32a, when the push-out operation of the embolization material .10 by the delivery pusher 30 is completed, the handle portion 32 can be easily pulled out simultaneously in the inserted state in accordance with a detachment operation of the embolization material loading catheter 20, thereby simplifying a detachment operation. The delivery pusher 30 may be pulled out from the embolization material loading catheter 20 before the detachment operation of the embolization material loading catheter 20.

The handle portion 32 may be configured such that the large-diameter head portion 32a can be fitted to the proximal side of the proximal hub 23 when the handle portion 32 is inserted into the embolization material loading catheter 20. With such a configuration, when the embolization material loading catheter 20 is detached, the delivery pusher 30 can be reliably pulled out without being detached from the embolization material loading catheter 20.

A constituent material for the pusher body 31 is not particularly limited as long as the constituent material is a material having appropriate hardness and flexibility such that the embolization material 10 can be conveyed. As examples of the constituent material for the pusher body 31, a resin material such as a polymer material including polyolefins (for example, polyethylene, polypropylene, polybutene, an ethylene-propylene copolymer, an ethylene-vinyl acetate copolymer, an ionomer, or a mixture of two or more types thereof), a polyolefin elastomer, a crosslinked polyolefin, polyvinyl chloride, polyamide, a polyamide elastomer, polyester, polyester elastomer, polyurethane, polyurethane elastomer, a fluorous resin such as ETFE, polycarbonate, polystyrene, polyacetal, polyimide, polyetherimide, and aromatic polyether ketone, or a mixture thereof, and a metal material such as a shape memory alloy, stainless steel, tantalum, titanium, platinum, gold, or tungsten can be suitably used.

### <Delivery System>

Next, the delivery system 200 according to the present embodiment will be described. As shown in FIG. 1, the delivery system 200 according to the present embodiment includes, in addition to the medical instrument set 100, the delivery catheter 40 to and from which the embolization material loading catheter 20 is attached and detached while indwelling in the body lumen.

The delivery catheter 40 can also use, for example, an existing catheter that can indwell in the body lumen. Therefore, in the delivery system 200 according to the present embodiment, the medical instrument set 100 and the delivery catheter 40 can be sold as a set and supplied to a market, but even when only the medical instrument set 100 is sold and supplied to the market, an existing catheter can be used as the delivery catheter 40 to function as the delivery system 200.

### (Delivery Catheter)

The delivery catheter 40 includes, for example, the sheath 41 formed of an elongated tubular member in which a hole (the sheath lumen 42) penetrating from an opening portion on a distal side to an opening portion on a proximal side along an axial direction is formed, and indwells in the body lumen and functions as an introduction passage for delivering the embolization material 10 into the aneurysm. A main body 51 of an insertion assisting member 50, which will be described later, can be inserted through the sheath 41 over a total length of the sheath 41. Therefore, a length of the sheath 41 in the axial direction is set to be at least shorter than a length of the main body 51 of the insertion assisting member 50.

As shown in FIG. 5, the inner diameter of the sheath lumen 42 is designed to be substantially equal to the inner diameter of the loading lumen 22. Accordingly, the embolization material 10 can be smoothly moved from the loading lumen 22 to the sheath lumen 42 when the embolization material loading catheter 20 and the delivery catheter 40 are in the connected state by the engagement portion 60.

A constituent material for the sheath 41 is not particularly limited as long as the constituent material is flexible and rigid enough to follow the bent shape of the body lumen such as meandering and bending. As examples of the constituent material for the sheath 41, a resin material such as a polymer material including polyolefins (for example, polyethylene, polypropylene, polybutene, an ethylene-propylene copolymer, an ethylene-vinyl acetate copolymer, an ionomer, or a mixture of two or more types thereof), a polyolefin elastomer, a crosslinked polyolefin, polyvinyl chloride, polyamide, a polyamide elastomer, polyester, polyester elastomer, polyurethane, polyurethane elastomer, a fluorous resin, polycarbonate, polystyrene, polyacetal, polyimide, polyetherimide, and aromatic polyether ketone, or a mixture thereof can be suitably used.

The delivery catheter 40 includes the sheath hub 43 interlocked to the proximal side of the sheath 41, and the flexible tube 44 having one end connected to the proximal side of the sheath hub 43 and the other end connected to a three-way stopcock 45.

The sheath hub 43 is an intermediate member that includes a communication passage 43a that allows communication between the sheath lumen 42 and the tube 44 and between the loading lumen 22 and the sheath lumen 42, and allows a fluid (such as physiological saline for priming) flowing from the three-way stopcock 45 to flow to the sheath 41 via the tube 44 and to guide the embolization material 10 pushed out from the embolization material loading catheter 20 into the sheath lumen 42. The insertion assisting member 50 is inserted into the sheath hub 43 when the delivery catheter 40 indwells in the body lumen.

A constituent material for the sheath hub 43 may be the same as the material exemplified as the constituent material for the proximal hub 23 described above.

The communication passage 43a includes the communication distal portion 43b having an inner diameter substantially equal to the inner diameter of the sheath lumen 42, and the communicating enlarged-diameter portion 43c extending from the communication distal portion 43b in a proximal direction and serving as an internal space having a diameter larger than that of the communication distal portion 43b. The tapered portion 43d increased in diameter in the proximal direction from an opening portion on the proximal side of the communication distal portion 43b is provided in a portion of the communicating enlarged-diameter portion 43c connected to the communication distal portion 43b.

In the mounted state between the embolization material loading catheter 20 and the delivery catheter 40, a distal end of the loading lumen 22 is inserted so as to be adjacent to the opening portion on the proximal side of the communication distal portion 43b. Accordingly, when the embolization material 10 is pushed out from the embolization material loading catheter 20 to the delivery catheter 40, the embolization material 10 can be sent out from the loading lumen 22 to the sheath lumen 42 without being exposed to the outside.

The insertion state of the insertion portion 27a is preferably such that the inner surface of the tapered portion 43d and the distal contact portion 27b come into contact with each other so that the loading lumen 22 and the sheath lumen 42 are aligned in the axial direction. Accordingly, since a distal end of the insertion portion 27a and the opening portion on the proximal side of the communication distal portion 43b are connected along the axial direction, the pushed-out embolization material 10 is prevented from being damaged (bent or crushed on the distal side) due to abutment of the embolization material 10 against the inner wall surface of the sheath hub 43, and the loading lumen 22 and the sheath lumen 42 are communicated with each other so as not to intersect with a space inside the communicating enlarged-diameter portion 43c, thereby preventing the embolization material 10 from being erroneously inserted into other spaces within the sheath hub 43 (for example, erroneously entering into the tube 44 connected to the sheath hub 43).

As shown in FIGS. 3 and 4, the proximal side of the sheath hub 43 is provided with the first engagement portion 48 to be engaged with the second engagement portion 28 provided at the distal connection portion 27 of the embolization material loading catheter 20.

For example, the first engagement portion 48 may be configured by the groove portion 48a into which the engagement claws 28a of the second engagement portion 28 described above are fitted. The groove portion 48a is provided along the circumferential direction on the outer circumferential surface of the sheath hub 43 on the proximal side in FIG. 3, and is provided along a circumferential direction on the inner wall surface of the inserting portion 48b formed in a substantially central portion of the end surface of the sheath hub 43 on the proximal side in FIG. 4.

The one end of the tube 44 is interlocked to the proximal side of the sheath hub 43, and the other end is interlocked to the port 46 of the three-way stopcock 45. The tube 44 is a conduit through which a liquid such as physiological salt solution flowing out from a priming syringe (not shown) interlocked to the port 46 flows. A constituent material for the tube 44 may be the same as the material exemplified as the constituent material for the tube 24 described above.

The three-way stopcock 45 communicates with the sheath lumen 42 of the sheath 41 via the communication passage 43a of the sheath hub 43 and the tube 44. In addition to a proximal side of the tube 44, a priming syringe for priming the sheath lumen 42 of the sheath 41 and a liquid agent injection syringe for injecting a contrast agent, a drug, or the like can be connected to the port 46 of the three-way stopcock 45.

A hemostasis valve 47 is attached into the proximal side of the sheath hub 43. As the hemostasis valve 47, for example, a substantially elliptical film-shaped (disk-shaped) valve body made of silicone rubber, latex rubber, butyl rubber, or isoprene rubber which is an elastic member may be used. When the embolization material loading catheter 20 is mounted to the delivery catheter 40, at least the distal contact portion 27b of the insertion portion 27a passes through the hemostasis valve 47 and is inserted into the communicating enlarged-diameter portion 43c.

Here, an example of dimensions of each device constituting the delivery system 200 according to the present embodiment will be described. Numerical values shown below are merely examples, and the present invention is not limited thereto.

In the delivery system 200 according to the present embodiment, when the delivery catheter 40 is a catheter having an outer diameter of 6 Fr (an inner diameter of 1.8 mm) and a surgical method to be applied is endoleak embolization for stent graft interpolation of an abdominal aortic aneurysm (AAA), the outer diameter of the embolization material 10 can be 0.4 to 1.9 mm (preferably about 1.6 mm), and the inner diameter of the embolization material loading catheter 20 can be 1.0 to 1.8 mm (preferably about 1.8 mm), which is equal to the inner diameter of the delivery catheter 40. A body length of the catheter body 21 of the embolization material loading catheter 20 can be 30 to 105 cm (preferably about 42 cm), a body length of the sheath 41 of the delivery catheter 40 can be 39 to 90 cm (preferably about 47 cm), and a body length of the pusher body 31 of the delivery pusher 30 can be 79 to 205 cm (preferably about 96 cm). The total length of the embolization material 10 is appropriately determined depending on a size of the aneurysm, and may be in a range of 30 to 100 cm (preferably about 40 cm) from the viewpoint of ease of loading into the embolization material loading catheter 20 and shortening the procedure time.

### <Embolization material Delivery Medical System>

Next, the embolization material delivery medical system 300 according to the present embodiment will be described. As shown in FIG. 2, the embolization material delivery medical system 300 according to the present embodiment includes, in addition to the delivery system 200, the insertion assisting member 50 that delivers the delivery catheter 40 into the body lumen.

### (Insertion Assisting Member)

The insertion assisting member 50 is an auxiliary tool that is provided with a guide wire lumen 52 that is inserted from a distal side to a proximal side along an axial direction of the main body 51, and assists insertion when the delivery catheter 40 is delivered into the aneurysm along a guide wire inserted into the body lumen in advance.

The insertion assisting member 50 is inserted into and assembled to the delivery catheter 40 in order to prevent bending or the like when the delivery catheter 40 is inserted into the body lumen. The guide wire lumen 52 has an inner diameter smaller than that of the sheath lumen 42 of the delivery catheter 40. Therefore, when the delivery catheter 40 is delivered into the aneurysm, axial deviation of the delivery catheter 40 with respect to the guide wire can be reduced, which makes delivery easier.

A constituent material for the insertion assisting member 50 is not particularly limited as long as the constituent material is harder and more flexible than a constituent material for the delivery catheter 40. As an example of the constituent material for the insertion assisting member 50, polyolefins such as polyethylene or polypropylene, polyester such as polyamide or polyethylene terephthalate, a fluorous resin such as ETFE, a resin material such as PEEK (polyether ether ketone) or polyimide, a metal material such as a shape memory alloy, stainless steel, tantalum, titanium, platinum, gold, or tungsten can be suitably used.

### [Operations]

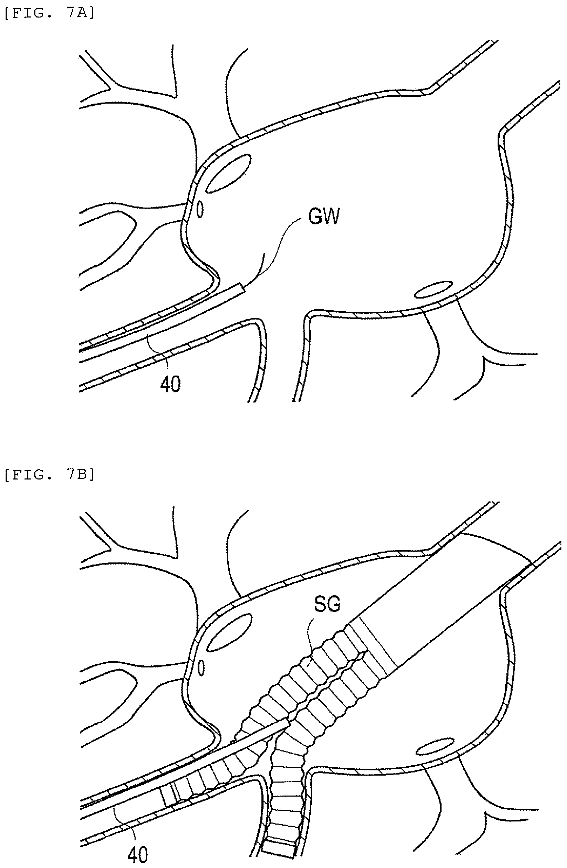

Next, an operation of the embolization material delivery medical system 300 according to the present embodiment will be described with reference to FIGS. 7A to 7G as appropriate. Here, an operation example when the embolization material delivery medical system 300 is applied to the endoleak embolization for the stent graft interpolation of the abdominal aortic aneurysm (AAA) is described, and configuration examples of the engagement portion 60 are shown in FIGS. 3A and 3B. In each drawing, the inside of the aneurysm is represented by "A", the inside of the blood vessel is represented by "V", the outside of the body is represented by "O", and installation positions of the devices of the embolization material delivery medical system 300 are expressed so as to be systematically grasped.

First, as a preparation step before surgery, as shown in FIG. 7A, the surgeon percutaneously inserts, from a limb of a patient serving as a puncture site into the body lumen via an introducer (for example, a member indicated by a two-dot chain line in FIG. 7A), the sheath 41 of the delivery catheter 40 into which the insertion assisting member 50 is inserted, and delivers a distal end opening 41a of the delivery catheter 40 to the abdominal aortic aneurysm. When the distal end opening 41a is delivered into the aneurysm, the insertion assisting member 50 is removed. The delivery catheter 40 may be delivered to an aneurysm lesion by using the guide wire inserted in the aneurysm in advance without using the insertion assisting member 50.

Next, as shown in FIG. 7B, the surgeon inserts, into the body lumen via the introducer, a catheter (a stent graft device) into which a stent graft SG is compressed and inserted, and moves the catheter to the aneurysm lesion using the guide wire inserted into the aneurysm in advance. Thereafter, the stent graft SG is expanded from the catheter and indwells in the target lesion. Accordingly, as shown in FIG. 7B, the delivery catheter 40 is inserted between a leg of the stent graft SG and a blood vessel wall, a distal portion of the delivery catheter 40 is inserted between the stent graft SG and a blood vessel wall of the aneurysm, that is, into the aneurysm, and the delivery catheter 40 indwells in the body lumen with the distal end opening 41a positioned in the aneurysm.

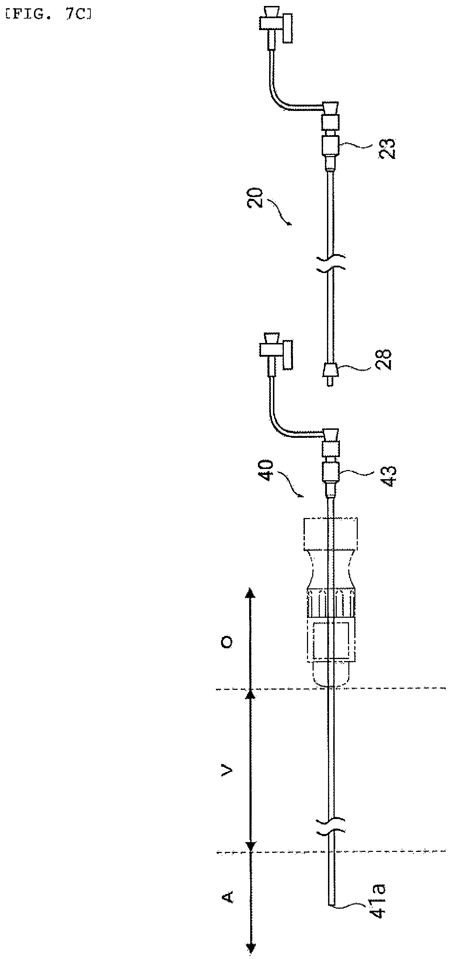

As shown in FIG. 7C, the embolization material loading catheter 20 loaded with the embolization material 10 is prepared. FIG. 7C shows a state before the embolization material loading catheter 20 is mounted to the delivery catheter 40.

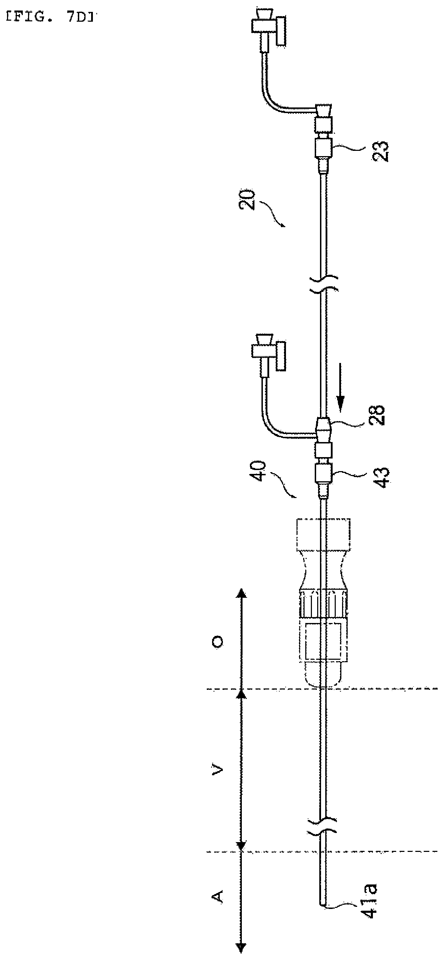

When the delivery catheter 40 indwells, the surgeon mounts the distal connection portion 27 of the embolization material loading catheter 20 to a proximal end of the sheath hub 43 of the delivery catheter 40, as shown in FIG. 7D. At this time, as shown in FIG. 3B, the distal contact portion 27b of the dista1 connection portion 27 is inserted into the communicating enlarged-diameter portion 43c of the sheath hub 43 and comes into contact with the inner surface of the tapered portion 43d. Accordingly, the distal end of the loading lumen 22 is adjacent to the communication distal portion 43b, and an axis of the loading lumen 22 and an axis of the sheath lumen 42 are aligned.

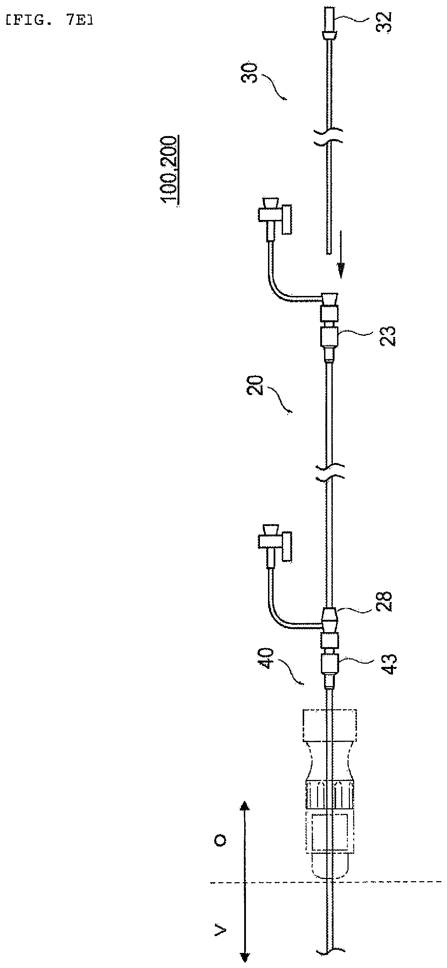

Next, the surgeon inserts a distal end of the pusher body 31 from the proximal side of the proximal hub 23 while gripping the handle portion 32, as shown in FIG. 7E. A distal end of the delivery pusher 30 inserted from the proximal hub 23 comes into contact with a proximal end of the embolization material 10 loaded in the embolization material loading catheter 20, and the embolization material 10 is pushed out and moved to the sheath lumen 42 of the delivery catheter 40 by the push-out operation.

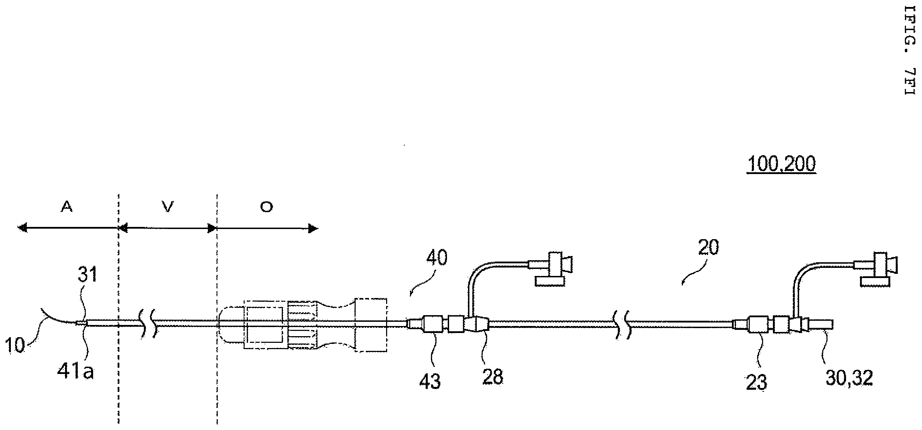

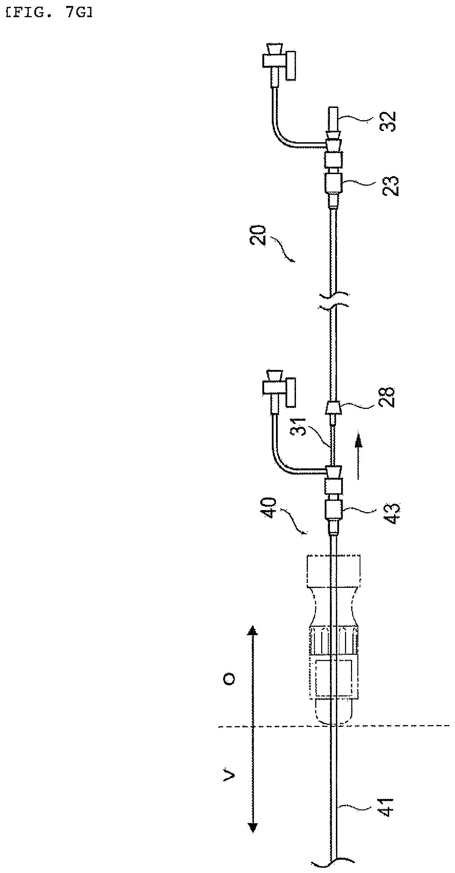

As shown in FIG. 7F, the surgeon pushes out the delivery pusher 30 inserted from the proximal hub 23 to push out the embolization material 10 from the sheath lumen 42 into the aneurysm. Thereafter, the surgeon detaches the emptied embolization material loading catheter 20 together with the delivery pusher 30 from the delivery catheter 40, as shown in FIG. 7G. The delivery pusher 30 can be detached from the delivery catheter 40 while being inserted into the embolization material loading catheter 20. Thus, a first insertion operation of the embolization material 10 into the aneurysm is completed. In the insertion operation, the delivery pusher 30 may be pulled out from the embolization material loading catheter 20 before the detachment operation of the embolization material loading catheter 20. Such a series of embolization material indwelling operations shown in FIGS. 7C to 7G are repeated until a required amount of embolization material 10 is loaded into the aneurysm. The required amount is calculated as a value obtained by calculating a volume of the aneurysm based on CT data about the patient and subtracting the volume of the stent graft SG when the stent graft SG is expanded in the aneurysm from the calculated value.

After the required amount of embolization material 10 has indwelled in the aneurysm, the surgeon pulls out the delivery catheter 40 from the aneurysm and the body lumen. At this time, the delivery catheter 40 may be pulled out from the aneurysm and the body lumen in a state where the embolization material loading catheter 20 is mounted to the delivery catheter 40 and the delivery pusher 30 is inserted into the delivery catheter 40. Before the delivery catheter 40 is pulled out from the aneurysm and the body lumen, the delivery pusher 30 may be pulled out from the delivery catheter 40 while the embolization material loading catheter 20 is detached from the delivery catheter 40. Before the delivery catheter 40 is pulled out from the aneurysm and the body lumen, the delivery pusher 30 may be pulled out from the delivery catheter 40 and the embolization material loading catheter 20, and then the embolization material loading catheter 20 may be detached from the delivery catheter 40. In any case, the introducer remains indwelling in the body lumen for additional expansion of the stent graft SG by a balloon after the embolization material 10 indwells, an imaging operation, and the like.

Thereafter, the embolization material 10 indwelling in the aneurysm comes into contact with a liquid such as blood in the aneurysm and gradually swells, and the completely expanded embolization material 10 fills a space between an inner surface of the aneurysm and an outer surface of the stent graft, thereby closing the aneurysm. Accordingly, the aneurysm is prevented from rupture.

As described above, the embolization material delivery medical system 300 including the medical instrument set 100 and the delivery system 200 according to the present embodiment can deliver the embolization material 10 into the aneurysm only in three procedures as shown in FIGS. 7A to 7C. Therefore, the number of procedure steps is reduced, and the procedure time can be shortened as compared with the indirect insertion method which can be assumed as the delivery method of the embolization material 10.

### [Modification]

The embodiment described above can also be modified to have the following configuration.

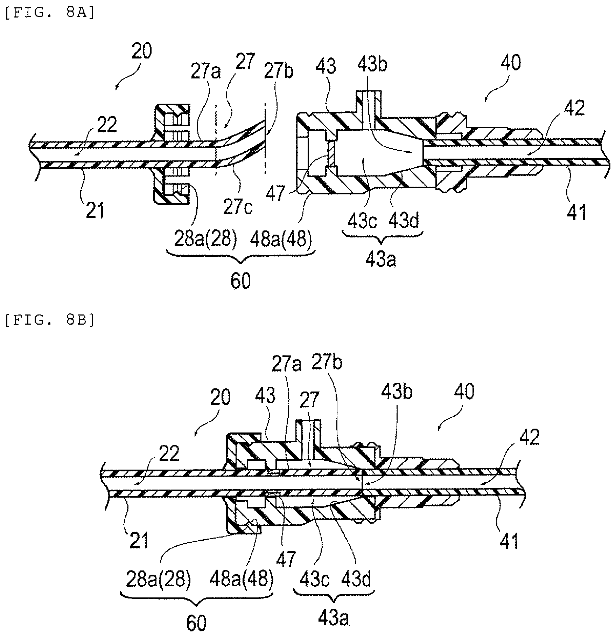

FIG. 8 shows another configuration example of the insertion portion 27a of the distal connection portion 27 in the embolization material loading catheter 20. As shown in FIG. 8A, the insertion portion 27a according to a modification includes a bending portion 27c. The bending portion 27c forms at least a part of the insertion portion 27a, and has a shape that is gradually bent in a direction away from a central axis of the loading lumen 22 (a radial direction with respect to the central axis of the loading lumen 22) toward a distal side of the loading lumen 22 (the distal contact portion 27b). The bending portion 27c may be provided, for example, in a region partitioned by a two-dot chain line shown in FIG. 8A in the insertion portion 27a of the embolization material loading catheter 20. In the shown example, a part of an intermediate portion located between a proximal end of the insertion portion 27a and the dista1 contact portion 27b (an opening portion of the distal end) is formed by the bending portion 27c, and is bent in a bent shape.

As shown in FIG. 8B, when the bending portion 27c is inserted into the communicating enlarged-diameter portion 43c of the sheath hub 43, the bent shape of the bending portion 27c is corrected into a substantially straight state. In the mounted state where the embolization material loading catheter 20 is mounted to the delivery catheter 40, the bending portion 27c is corrected such that the axis of the loading lumen 22 and the axis of the sheath lumen 42 of the delivery catheter 40 are aligned in a state where the distal contact portion 27b is in contact with the inner surface of the tapered portion 43d of the sheath hub 43. That is, when the bending portion 27c is inserted into the communicating enlarged-diameter portion 43c, the bending portion 27c comes into contact with an inner wall or the like of the communicating enlarged-diameter portion 43c, whereby the bent shape thereof is corrected into a substantially straight shape. In particular, the distal contact portion 27b comes into contact with the inner wall of the communicating enlarged-diameter portion 43c, and a proximal portion of the insertion portion 27a comes into contact with the hemostasis valve 47 of the sheath hub or the inner wall of the opening portion on a proximal side of the sheath hub, whereby the bending portion 27c is corrected into a substantially straight shape.

The bending portion 27c has a bent shape with a predetermined curvature smoothly deformed in an arch shape from the proximal end of the insertion portion 27a to the distal contact portion 27b (an opening portion of the distal end) or from the intermediate portion of the insertion portion 27a to the distal contact portion 27b (an opening portion of the distal end), and also has a bent shape such as a substantially bent shape bent from a certain starting point.

A constituent material for the bending portion 27c is not particularly limited as long as the constituent material has flexibility such that the bending portion 27c keeps a bent state before being inserted into the sheath hub 43, comes into contact with the inner wall or the like of the communicating enlarged-diameter portion 43c and can be elastically deformed into a substantially straight state after being inserted into the sheath hub 43. Since the bending portion 27c only needs to have lower rigidity than the sheath hub 43, in order to produce a difference in rigidity, the bending portion 27c may be structured by changing the constituent material, or by changing a thickness of the bending portion 27c when the same material is employed.

Thus, by providing the bending portion 27c in at least a part of the insertion portion 27a, it is possible to prevent the embolization material 10 loaded in the loading lumen 22 from erroneously falling off from a distal end of the distal connection portion 27. When the bending portion 27c is inserted into the sheath hub 43, the bent shape of the bending portion 27c is corrected to a substantially straight state, so that the axis of the loading lumen 22 and the axis of the sheath lumen 42 are aligned. Therefore, even if the insertion portion 27a is partially bent, the insertion of the embolization material 10 is not hindered.

### [Functions and Effects]

As described above, the medical instrument set 100 according to the present embodiment is configured to deliver the embolization material 10 into an aneurysm via the delivery catheter 40. The delivery catheter 40 includes the sheath 41 that includes the sheath lumen 42 and the distal end opening 41a that communicates with the sheath lumen 42, and the sheath hub 43 that includes the communication passage 43a communicating with a proximal end of the sheath lumen 42 and is provided on the proximal side of the sheath 41. The medical instrument set 100 includes: the embolization material loading catheter 20 that includes the catheter body 21 including the loading lumen 22 having an open distal end, and the proximal hub 23 including the insertion passage 23a communicating with a proximal end of the loading lumen 22, and in which the embolization material 10 is loaded in the loading lumen 22; and the delivery pusher 30 that includes the elongated pusher body 31 insertable into the loading lumen 22 through the insertion passage 23a of the proximal hub 23 of the embolization material loading catheter 20. The catheter body 21 includes the distal connection portion 27 attachable to and detachable from the sheath hub 43 of the delivery catheter 40 on the distal side of the catheter body 21. The pusher body 31 has a body length longer than a distance from the proximal end of the insertion passage 23a of the proximal hub 23 to the distal end opening 41a of the sheath 41 of the delivery catheter 40 in the mounted state where the distal connection portion 27 of the catheter body 21 is mounted to the sheath hub 43 of the delivery catheter 40, and when the pusher body 31 is inserted from the insertion passage 23a of the proximal hub 23 in the mounted state, the embolization material 10 is passed through the sheath lumen 42 of the delivery catheter 40 and is pushed out from the distal end opening 41a of the delivery catheter 40 into the aneurysm.

The pusher body 31 of the delivery pusher 30 is formed so as to be longer than the distance from the proximal end of the insertion passage 23a of the proximal hub 23 to the distal end opening 41a of the sheath 41 of the delivery catheter 40 in the mounted state between the embolization material loading catheter 20 and the delivery catheter 40. Therefore, in the mounted state where the embolization material loading catheter 20 is mounted to the delivery catheter 40, by inserting the delivery pusher 30 from the proximal hub 23, the embolization material 10 loaded in the loading lumen 22 can be pushed out into the aneurysm by a single push-out operation. Therefore, the number of procedure steps is reduced. In addition, since it is only necessary to mount the embolization material loading catheter 20 to the sheath hub 43 of the delivery catheter 40, the procedure can be simplified, and as a result, the procedure time can be shortened.

In the medical instrument set 100 according to the present embodiment, preferably, the inner diameter of the loading lumen 22 may be substantially equal to the inner diameter of the sheath lumen 42.

With such a configuration, the outer diameter of the embolization material 10 loaded into the loading lumen 22 can be made substantially equal to the inner diameters of the loading lumen 22 and the sheath lumen 42. Therefore, the diameter size of the embolization material 10 can be made large without being made small in accordance with the loading lumen 22 as in the direct insertion method and the indirect insertion method, so that the number of inserted embolization material 10 can be reduced and the procedure time can be shortened.

In the medical instrument set 100 according to the present embodiment, preferably, the distal connection portion 27 of the catheter body 21 may include the insertion portion 27a that is inserted into the sheath hub 43 of the delivery catheter 40 in the mounted state.

With such a configuration, when the embolization material loading catheter 20 is mounted to the delivery catheter 40, the insertion portion 27a can be inserted into the sheath hub 43, so that the embolization material 10 can be pushed out from the loading lumen 22 to the sheath lumen 42 via the sheath hub 43 without being exposed to the outside.

In the medical instrument set 100 according to the present embodiment, preferably, the delivery pusher 30 may include the handle portion 32 provided on the proximal side of the pusher body 31, and the maximum outer diameter of the handle portion 32 may be larger than an inner diameter of the insertion passage 23a of the proximal hub 23.

With such a configuration, when the delivery pusher 30 is inserted into the embolization material loading catheter 20, since a distal end of the handle portion 32 (the large-diameter head portion 32a) is not inserted into the insertion passage 23a of the proximal hub 23, an insertion length of an insertion pusher of the delivery pusher 30 can be limited. Since the handle portion 32 does not enter the proximal hub 23, when the push-out operation of the embolization material 10 by the delivery pusher 30 is completed, the delivery pusher 30 can be easily pulled out simultaneously with the detachment operation of the embolization material loading catheter 20.

The delivery system 200 according to the present embodiment includes the medical instrument set 100 and the delivery catheter 40, and is configured such that, in the mounted state, the embolization material 10 is passed through the sheath lumen 42 of the delivery catheter 40 by the delivery pusher 30 inserted from the insertion passage 23a of the proximal hub 23 and is pushed out from the distal end opening 41a of the delivery catheter 40 into the aneurysm.

When the distal connection portion 27 of the embolization material loading catheter 20 is engaged with the sheath hub 43 of the delivery catheter 40 indwelling in the body lumen to be in the mounted state, the loading lumen 22 and the sheath lumen 42 are in communication with each other through the communication passage 43a of the sheath hub 43. Therefore, by inserting the delivery pusher 30 from the proximal hub 23, the embolization material 10 loaded in the loading lumen 22 can be pushed out into the aneurysm by a single push-out operation.

The delivery system 200 according to the present embodiment may be configured such that, preferably, the delivery catheter 40 includes the first engagement portion 48 including the groove portion 48a provided on the proximal side of the sheath hub 43, the embolization material loading catheter 20 includes the second engagement portion 28 including the engagement claws 28a to be engaged with the first engagement portion 48 in the distal connection portion 27, and the first engagement portion 48 and the second engagement portion 28 are engaged with each other in the mounted state to allow communication between the loading lumen 22 and the sheath lumen 42.

In the delivery system 200, since the second engagement portion 28 including the plurality of engagement claws 28a provided in the delivery catheter 40 is engaged with the groove portion 48a, which is the first engagement portion 48 provided in the embolization material loading catheter 20, the embolization material loading catheter 20 is attachable to and detachable from the delivery catheter 40. Therefore, after the embolization material 10 is pushed out into the aneurysm by the push-out operation, by releasing an engagement state between the first engagement portion 48 and the second engagement portion 28, it is possible to easily restart an indwelling operation of the embolization material 10 including the push-out operation by mounting another embolization material loading catheter 20 in which the embolization material 10 has been loaded or reloading the embolization material 10 into the embolization material loading catheter 20 which has been detached.

The delivery system 200 according to the present embodiment may be configured such that, preferably, the communication passage 43a of the sheath hub 43 of the delivery catheter 40 includes the communication distal portion 43b having the inner diameter substantially equal to the inner diameter of the sheath lumen 42, and the communicating enlarged-diameter portion 43c extending from the communication distal portion 43b in the proximal direction and having a diameter larger than that of the communication distal portion 43b, and the distal connection portion 27 of the catheter body 21 includes the distal end of the loading lumen 22 and includes the insertion portion 27a to be inserted into the communicating enlarged-diameter portion 43c of the sheath hub 43 in the mounted state.

Since the insertion portion 27a is inserted such that the loading lumen 22 and the sheath lumen 42 are aligned in the axial direction in the connected state between the embolization material loading catheter 20 and the delivery catheter 40, the embolization material 10 is pushed out to the sheath lumen 42 from the loading lumen 22 without being exposed to the outside through the sheath hub 43.

The delivery system 200 according to the present embodiment may be configured such that, preferably, the communicating enlarged-diameter portion 43c includes the tapered portion 43d enlarged in diameter in the proximal direction, and the insertion portion 27a of the distal connection portion 27 includes the distal contact portion 27b that comes into contact with the inner surface of the tapered portion 43d such that the axis of the loading lumen 22 and the axis of the sheath lumen 42 of the delivery catheter 40 are aligned in the mounted state.

When the insertion portion 27a is inserted into the communicating enlarged-diameter portion 43c, if the distal contact portion 27b comes into contact with the tapered portion 43d, the loading lumen 22 and the sheath lumen 42 communicate with each other so as not to intersect with other spaces including the space of the communicating enlarged-diameter portion 43c. Therefore, when the embolization material 10 is pushed out from the embolization material loading catheter 20 into the delivery catheter 40, the embolization material 10 is prevented from being damaged (bent or crushed on the distal side) due to abutment of the embolization material 10 against the inner wall surface of the sheath hub 43, is prevented from being erroneously inserted into other spaces within the sheath hub 43 (for example, erroneously entering into the tube 44 connected to the sheath hub 43), and is reliably delivered into the aneurysm.

The delivery system 200 according to the present embodiment may be configured such that, preferably, the insertion portion 27a includes the bending portion 27c that is gradually bent away from the axial direction of the loading lumen 22 toward the distal end of the loading lumen 22 (distal contact portion 27b), and when the insertion portion 27a is inserted into the sheath hub 43, the bent shape of the bending portion 27c is corrected, and in the mounted state, the distal contact portion 27b and the inner surface of the tapered portion 43d come into contact with each other, and the axis of the loading lumen 22 and the axis of the sheath lumen 42 of the delivery catheter 40 are aligned.

By providing the bending portion 27c in at least a part of the insertion portion 27a, it is possible to prevent the embolization material 10 loaded in the loading lumen 22 from erroneously falling off from the distal end of the distal connection portion 27. When the bending portion 27c is inserted into the sheath hub 43, the bent shape thereof is corrected to a substantially straight state. Therefore, since the axis of the loading lumen 22 and the axis of the sheath lumen 42 are aligned in the mounted state between the embolization material loading catheter 20 and the delivery catheter 40, the insertion of the embolization material 10 is not hindered.

The delivery system 200 according to the present embodiment may be configured such that, preferably, the insertion portion 27a of the distal connection portion 27 is inserted into the communicating enlarged-diameter portion 43c such that the distal end of the loading lumen 22 is adjacent to the communication distal portion 43b in the mounted state.

Since the insertion portion 27a is inserted into the communicating enlarged-diameter portion 43c such that the distal end of the loading lumen 22 is adjacent to the communication distal portion 43b, the embolization material 10 pushed out from the embolization material loading catheter 20 to the delivery catheter 40 is prevented from being damaged by abutting against the inner wall surface of the sheath hub 43 or from being erroneously inserted into other spaces within the sheath hub 43.

In the delivery system 200 according to the present embodiment, preferably, the catheter body 21 of the embolization material loading catheter 20 may be configured to have rigidity higher than that of the sheath 41 of the delivery catheter 40.

In the delivery system 200, since the embolization material loading catheter 20 is not used by being inserted into a sheath of the delivery catheter 40 as in the direct insertion method, the embolization material loading catheter 20 does not require flexibility to follow the bent shape of the body lumen during insertion, and the rigidity of the catheter body 21 can be made higher than that of the sheath 41. Therefore, the embolization material 10 is prevented from being damaged during packaging or unpacking.

The embolization material delivery medical system 300 according to the present embodiment includes: the delivery system 200; and the elongated insertion assisting member 50 to be assembled into the sheath lumen 42 of the delivery catheter 40 in order to assist delivery of the delivery catheter 40 into the aneurysm. The insertion assisting member 50 includes the guide wire lumen 52 that penetrates from a distal end to a proximal end and has a diameter smaller than that of the sheath lumen 42 of the delivery catheter 40.

With such a configuration, when the delivery catheter 40 is to be inserted into the body lumen, since the insertion assisting member 50 is assembled, the delivery catheter 40 can be inserted into the body lumen smoothly.

### Reference Signs List

- 10: embolization material
- 20: embolization material loading catheter
- 21: catheter body
- 22: loading lumen
- 23: proximal hub (23a insertion passage)
- 24: tube
- 25: three-way stopcock
- 26: port
- 27: distal connection portion (27a insertion portion,
- 27b: distal contact portion, 27c bending portion)
- 28: second engagement portion (28a engagement claw)
- 30: delivery pusher
- 31: pusher body
- 32 32b: handle portion (32a large-diameter head portion, small-diameter handle portion)
- 40: delivery catheter
- 41: sheath (41a distal end opening)
- 42: sheath lumen
- 43: sheath hub (43a communication passage, 43b communication distal portion, 43c communicating enlarged-diameter portion, 43d tapered portion)
- 44: tube
- 45: three-way stopcock
- 46: port
- 47: hemostasis valve
- 48: first engagement portion (48a groove portion, 48b inserting portion)
- 50: insertion assisting member
- 51: main body
- 52: guide wire lumen
- 60: engagement portion
- 100: medical instrument set
- 200: delivery system
- 300: embolization material delivery medical system
- GW: guide wire
- SG: stent graft.

## Claims

1. A delivery system (200) comprising:
a medical instrument set (100) for delivering an embolization material (10) into an aneurysm via a delivery catheter (40), the delivery catheter (40) including a sheath (41) that includes a sheath lumen (42) and a distal end opening (41a) that communicates with the sheath lumen (42), and a sheath hub (43) that includes a communication passage (43a) communicating with a proximal end of the sheath lumen (42) and is provided on a proximal side of the sheath (41), and
the delivery catheter (40), wherein
the medical instrument set (100) comprising:
an embolization material loading catheter (20) that includes a catheter body (21) including a loading lumen (22) loaded with the embolization material (10) and having an open distal end, and a proximal hub (23) including an insertion passage (23a) communicating with a proximal end of the loading lumen (22); and
a delivery pusher (30) that includes an elongated pusher body (31) insertable into the loading lumen (22) through the insertion passage (23a) of the proximal hub (23) of the embolization material loading catheter (20), wherein
the embolization material loading catheter (20) includes a distal connection portion (27) attachable to and detachable from the sheath hub (43) of the delivery catheter (40) at a distal portion of the catheter body (21),
the pusher body (31) has a body length longer than a distance from a proximal end of the insertion passage (23a) of the proximal hub (23) to the distal end opening (41a) of the sheath (41) of the delivery catheter (40) in a mounted state where the distal connection portion (27) of the catheter body (21) is mounted to the sheath hub (43) of the delivery catheter (40), and when the pusher body (31) is inserted from the insertion passage (23a) of the proximal hub (23) in the mounted state, the embolization material (10) is passed through the sheath lumen (42) of the delivery catheter (40) and is pushed out from the distal end opening (41a) of the delivery catheter (40) into the aneurysm,
in the mounted state, the embolization material (10) is passed through the sheath lumen (42) of the delivery catheter (40) by the delivery pusher (30) inserted from the insertion passage (23a) of the proximal hub (23), and is pushed out from the distal end opening (41a) of the delivery catheter (40) into the aneurysm,
the communication passage (43a) of the sheath hub (43) of the delivery catheter (40) includes: a communication distal portion (43b) having an inner diameterequal to an inner diameter of the sheath lumen (42); and a communicating enlarged-diameter portion (43c) extending from the communication distal portion (43b) in a proximal direction and having a diameter larger than that of the communication distal portion (43b), and
the distal connection portion (27) of the catheter body (21) includes an insertion portion (27a) that includes a distal end of the loading lumen (22) and is inserted into the communicating enlarged-diameter portion (43c) of the sheath hub (43) in the mounted state,
**characterized in that**
the communicating enlarged-diameter portion (43c) has a tapered portion (43d) whose diameter is enlarged in the proximal direction, and
the insertion portion (27a) of the distal connection portion (27) includes a distal contact portion (27b) that comes into contact with an inner surface of the tapered portion (43d) such that an axis of the loading lumen (22) and an axis of the sheath lumen (42) of the delivery catheter (40) are aligned in the mounted state.

2. The delivery system (200) according to claim 1, wherein
an inner diameter of the loading lumen (22) is the same as an inner diameter of the sheath lumen (42).

3. The delivery system (200) according to claim 1 or 2, wherein
the distal connection portion (27) of the catheter body (21) includes an insertion portion (27a) inserted into a hub of the delivery catheter (40) in the mounted state.

4. The delivery system (200) according to any one of claims 1 to 3, wherein
the delivery pusher (30) includes a handle portion (32) provided on a proximal side of the pusher body (31), and
a maximum outer diameter of the handle portion (32) is larger than an inner diameter of the insertion passage (23a) of the proximal hub (23).

5. The delivery system (200) according to any one of claims 1 to 4, wherein
the delivery catheter (40) includes a first engagement portion (48) including a groove portion (48a) provided on a proximal side of the sheath hub (43),
the embolization material loading catheter (20) includes a second engagement portion (28) having an engagement claw (28a) that engages with the first engagement portion (48) at the distal connection portion (27), and
in the mounted state, the first engagement portion (48) and the second engagement portion (28) are engaged with each other to allow the loading lumen (22) communicate with the sheath lumen (42).

6. The delivery system (200) according to any one of claims 1 to 5, wherein
the insertion portion (27a) includes a bending portion (27c) that is gradually bent away from an axial direction of the loading lumen (22) toward the distal end of the loading lumen (22), and
the insertion portion (27a) is configured such that a bending shape of the bending portion (27c) is corrected when the insertion portion (27a) is inserted into the sheath hub (43), and in the mounted state, the distal contact portion (27b) and the inner surface of the tapered portion (43d) come into contact with each other, and the axis of the loading lumen (22) and the axis of the sheath lumen (42) of the delivery catheter (40) are aligned.

7. The delivery system (200) according to any one of claims 1 to 6, wherein
the insertion portion (27a) of the distal connection portion (27) is inserted into the communicating enlarged-diameter portion (43c) such that the distal end of the loading lumen (22) is adjacent to the communication distal portion (43b) in the mounted state.

8. The delivery system (200) according to any one of claims 1 to 7, wherein
the catheter body (21) of the embolization material loading catheter (20) has rigidity higher than that of the sheath (41) of the delivery catheter (40).

## Patentansprüche

1. Abgabesystem (200) umfassend:
einen Satz (100) mit medizinischen Instrumenten zum Einbringen eines Embolisationsmaterials (10) in ein Aneurysma über einen Einführungskatheter (40), wobei der Einführungskatheter (40) eine Hülle (41) umfasst, die ein Hüllenlumen (42) und eine distale Endöffnung (41a) aufweist, welche mit dem Hüllenlumen (42) in Verbindung steht, und einen Hüllennabe (43), die einen Verbindungsdurchgang (43a) aufweist, welcher mit einem proximalen Ende des Hüllenlumens (42) in Verbindung steht und an einer proximalen Seite der Hülle (41) vorgesehen ist, und
den Einführungskatheter (40), wobei
der Satz (100) mit medizinischen Instrumenten umfasst:
einen Katheter (20) zum Beladen mit Embolisationsmaterial, der einen Katheterkörper (21), welcher ein Beladungslumen (22), das mit dem Embolisationsmaterial (10) beladen ist, umfasst und ein offenes distales Ende aufweist, und eine proximale Nabe (23) umfasst, die einen Einführungskanal (23a) aufweist, welcher mit einem proximalen Ende des Beladungslumens (22) in Verbindung steht; und
einen Einführungsschieber (30), der einen langgestreckten Schieberkörper (31) umfasst, welcher durch den Einführungskanal (23a) von der proximalen Nabe (23) des Katheters (20) zum Beladen mit Embolisationsmaterial in das Beladungslumen (22) einführbar ist, wobei
der Katheter (20) zum Beladen mit Embolisationsmaterial einen distalen Verbindungsabschnitt (27) aufweist, welcher an einem distalen Abschnitt des Katheterkörpers (21) an der Hüllennabe (43) des Einführungskatheters (40) befestigbar und von dieser ablösbar ist,
der Schieberkörper (31) eine Körperlänge aufweist, die größer ist als ein Abstand von einem proximalen Ende des Einführungskanals (23a) der proximalen Nabe (23) bis zu der distalen Endöffnung (41a) der Hülle (41) des Einführungskatheters (40) in einem montierten Zustand, in welchem der distale Verbindungsabschnitt (27) des Katheterkörpers (21) an der Hüllennabe (43) von dem Einführungskatheter (40) montiert ist, und wenn der Schieberkörper (31) in dem montierten Zustand durch den Einführungskanal (23a) der proximalen Nabe (23) eingeführt wird, wird das Embolisationsmaterial (10) durch das Hüllenlumen (42) des Einführungskatheters (40) hindurchgeführt und aus der distalen Endöffnung (41a) des Einführungskatheters (40) in das Aneurysma herausgeschoben;
in dem montierten Zustand das Embolisationsmaterial (10) durch das Hüllenlumen (42) des Einführungskatheters (40) durch den Einführungsschieber (30), welcher von dem Einführungskanal (23a) der proximalen Nabe (23) eingeführt wurde, hindurchgeführt und aus der distalen Endöffnung (41a) des Einführungskatheters (40) in das Aneurysma herausgeschoben wird,
der Verbindungskanal (43a) von der Hüllennabe (43) des Einführungskatheters (40) umfasst: einen distalen Verbindungsabschnitt (43b), der einen Innendurchmesser aufweist, welcher einem Innendurchmesser des Hüllenlumens (42) entspricht; und einen in Verbindung stehenden Abschnitt (43c) mit vergrößertem Durchmesser, der sich von dem distalen Verbindungsabschnitt (43b) in einer proximalen Richtung erstreckt und einen Durchmesser aufweist, der größer ist als derjenige des distalen Verbindungsabschnitts (43b), und
der distale Verbindungsabschnitt (27) des Katheterkörpers (21) einen Einführungsabschnitt (27a) aufweist, der ein distales Ende des Beladungslumens (22) umfasst und in dem montierten Zustand in den damit in Verbindung stehenden Abschnitt (43c) mit vergrößertem Durchmesser von der Hüllennabe (43) eingeführt wird,
**dadurch gekennzeichnet, dass**
der in Verbindung stehende Abschnitt (43c) mit vergrößertem Durchmesser einen sich verjüngenden Abschnitt (43d) aufweist, dessen Durchmesser in der proximalen Richtung vergrößert ist, und
der Einführungsabschnitt (27a) des distalen Verbindungsabschnitts (27) einen distalen Kontaktabschnitt (27b) umfasst, der mit einer Innenfläche des sich verjüngenden Abschnitts (43d) derart in Kontakt kommt, dass eine Achse des Beladungslumens (22) und eine Achse des Hüllenlumens (42) des Einführungskatheters (40) in dem montierten Zustand ausgerichtet sind.

2. Abgabesystem (200) nach Anspruch 1, wobei
ein Innendurchmesser des Beladungslumens (22) zu einem Innendurchmesser des Hüllenlumens (42) identisch ist.

3. Abgabesystem (200) nach Anspruch 1 oder 2, wobei
der distale Verbindungsabschnitt (27) des Katheterkörpers (21) einen Einführungsabschnitt (27a) umfasst, der in dem montierten Zustand in eine Nabe des Einführungskatheters (40) eingeführt ist.

4. Abgabesystem (200) nach einem der Ansprüche 1 bis 3, wobei der Einführungsschieber (30) einen Griffabschnitt (32) aufweist, der an einer proximalen Seite des Schieberkörpers (31) vorgesehen ist, und
ein maximaler Außendurchmesser des Griffabschnitts (32) größer als ein Innendurchmesser des Einführungskanals (23a) der proximalen Nabe (23) ist.

5. Abgabesystem (200) nach einem der Ansprüche 1 bis 4, wobei der Einführungskatheter (40) einen ersten Eingriffsabschnitt (48) umfasst, der einen Nutabschnitt (48a) aufweist, welcher an einer proximalen Seite der Hüllennabe (43) vorgesehen ist,
der Katheter (20) zum Beladen mit Embolisationsmaterial einen zweiten Eingriffsabschnitt (28) umfasst, welcher eine Eingriffsklaue (28a) aufweist, die an dem distalen Verbindungsabschnitt (27) mit dem ersten Eingriffsabschnitt (48) in Eingriff steht, und
in dem montierten Zustand der erste Eingriffsabschnitt (48) und der zweite Eingriffsabschnitt (28) miteinander in Eingriff stehen, um eine Verbindung zwischen dem Beladungslumen (22) und dem Hüllenlumen (42) zu ermöglichen.

6. Abgabesystem (200) nach einem der Ansprüche 1 bis 5, wobei der Einführungsabschnitt (27a) einen Biegeabschnitt (27c) umfasst, der allmählich von einer axialen Richtung des Beladungslumens (22) weg in Richtung des distalen Endes des Beladungslumens (22) gebogen ist, und
der Einführungsabschnitt (27a) derart ausgebildet ist, dass eine gebogene Form des Biegeabschnitts (27c) korrigiert wird, wenn der Einführungsabschnitt (27a) in die Hüllennabe (43) eingeführt wird, und in dem montierten Zustand der distale Kontaktabschnitt (27b) und die Innenfläche des sich verjüngenden Abschnitts (43d) miteinander in Kontakt kommen und die Achse des Beladungslumens (22) und die Achse des Hüllenlumens (42) des Einführungskatheters (40) ausgerichtet sind.

7. Abgabesystem (200) nach einem der Ansprüche 1 bis 6, wobei der Einführungsabschnitt (27a) des distalen Verbindungsabschnitts (27) derart in den in Verbindung stehenden Abschnitt (43c) mit vergrößertem Durchmesser eingeführt wird, dass das distale Ende des Beladungslumens (22) in dem montierten Zustand an den distalen Verbindungsabschnitt (43b) angrenzt.

8. Abgabesystem (200) nach einem der Ansprüche 1 bis 7, wobei der Katheterkörper (21) des Katheters (20) zum Beladen mit Embolisationsmaterial eine höhere Steifigkeit aufweist als die Hülle (41) des Einführungskatheters (40).

## Revendications

1. Système d'administration (200) comprenant :
un jeu d'instruments médicaux (100) pour administrer un matériel d'embolisation (10) dans un anévrisme par le biais d'un cathéter d'administration (40), le cathéter d'administration (40) incluant une gaine (41) qui inclut une lumière de gaine (42) et une ouverture d'extrémité distale (41a) qui communique avec la lumière de gaine (42), et un embout femelle de gaine (43) qui inclut un passage de communication (43a) communiquant avec une extrémité proximale de la lumière de gaine (42) et est prévu sur un côté proximal de la gaine (41), et
le cathéter d'administration (40), dans lequel
le jeu d'instruments médicaux (100) comprend :
un cathéter de chargement de matériel d'embolisation (20) qui inclut un corps de cathéter (21) incluant une lumière de chargement (22) chargée avec le matériel d'embolisation (10) et ayant une extrémité distale ouverte, et un embout femelle proximal (23) incluant un passage d'insertion (23a) communiquant avec une extrémité proximale de la lumière de chargement (22) ; et
un poussoir d'administration (30) qui inclut un corps de poussoir allongé (31) pouvant être inséré dans la lumière de chargement (22) à travers le passage d'insertion (23a) de l'embout femelle proximal (23) du cathéter de chargement de matériel d'embolisation (20), dans lequel
le cathéter de chargement de matériel d'embolisation (20) inclut une partie de connexion distale (27) pouvant être fixée à, et séparable de, l'embout femelle de gaine (43) du cathéter d'administration (40) à une partie distale du corps de cathéter (21),
le corps de poussoir (31) présente une longueur de corps plus longue qu'une distance d'une extrémité proximale du passage d'insertion (23a) de l'embout femelle proximal (23) à l'ouverture d'extrémité distale (41a) de la gaine (41) du cathéter d'administration (40) dans un état monté où la partie de connexion distale (27) du corps de cathéter (21) est montée sur l'embout femelle de gaine (43) du cathéter d'administration (40), et lorsque le corps de poussoir (31) est inséré à partir du passage d'insertion (23a) de l'embout femelle proximal (23) dans l'état monté, le matériel d'embolisation (10) est passé au travers de la lumière de gaine (42) du cathéter d'administration (40) et est poussé hors de l'ouverture d'extrémité distale (41a) du cathéter d'administration (40) jusque dans l'anévrisme,
dans l'état monté, le matériel d'embolisation (10) est passé au travers de la lumière de gaine (42) du cathéter d'administration (40) par le poussoir d'administration (30) inséré à partir du passage d'insertion (23a) de l'embout femelle proximal (23), et est poussé hors de l'ouverture d'extrémité distale (41a) du cathéter d'administration (40) jusque dans l'anévrisme,
le passage de communication (43a) de l'embout femelle de gaine (43) du cathéter d'administration (40) inclut : une partie distale de communication (43b) ayant un diamètre intérieur égal à un diamètre intérieur de la lumière de gaine (42) ; et une partie de communication au diamètre élargi (43c) s'étendant de la partie distale de communication (43b) dans une direction proximale et ayant un diamètre plus grand que celui de la partie distale de communication (43b), et
la partie de connexion distale (27) du corps de cathéter (21) inclut une partie d'insertion (27a) qui inclut une extrémité distale de la lumière de chargement (22) et est insérée dans la partie de communication au diamètre élargi (43c) de l'embout femelle de gaine (43) dans l'état monté,
**caractérisé en ce que**
la partie de communication au diamètre élargi (43c) présente une partie conique (43d) dont le diamètre est élargi dans la direction proximale, et
la partie d'insertion (27a) de la partie de connexion distale (27) inclut une partie de contact distale (27b) qui vient en contact avec une surface intérieure de la partie conique (43d) de telle façon qu'un axe de la lumière de chargement (22) et un axe de la lumière de gaine (42) du cathéter d'administration (40) sont alignés dans l'état monté.

2. Système d'administration (200) selon la revendication 1, dans lequel
un diamètre intérieur de la lumière de chargement (22) est le même qu'un diamètre intérieur de la lumière de gaine (42).

3. Système d'administration (200) selon la revendication 1 ou 2, dans lequel
la partie de connexion distale (27) du corps de cathéter (21) inclut une partie d'insertion (27a) insérée dans un embout femelle du cathéter d'administration (40) dans l'état monté.

4. Système d'administration (200) selon l'une quelconque des revendications 1 à 3, dans lequel
le poussoir d'administration (30) inclut une partie de poignée (32) prévue sur un côté proximal du corps de poussoir (31), et
un diamètre extérieur maximal de la partie de poignée (32) est plus grand qu'un diamètre intérieur du passage d'insertion (23a) de l'embout femelle proximal (23).

5. Système d'administration (200) selon l'une quelconque des revendications 1 à 4, dans lequel
le cathéter d'administration (40) inclut une première partie d'engagement (48) incluant une partie de rainure (48a) prévue sur un côté proximal de l'embout femelle de gaine (43),
le cathéter de chargement de matériel d'embolisation (20) inclut une seconde partie d'engagement (28) ayant une griffe d'engagement (28a) qui s'engage avec la première partie d'engagement (48) sur la partie de connexion distale (27), et
dans l'état monté, la première partie d'engagement (48) et la seconde partie d'engagement (28) sont engagées l'une avec l'autre pour permettre à la lumière de chargement (22) de communiquer avec la lumière de gaine (42).

6. Système d'administration (200) selon l'une quelconque des revendications 1 à 5, dans lequel
la partie d'insertion (27a) inclut une partie de flexion (27c) qui est fléchie graduellement à distance d'une direction axiale de la lumière de chargement (22) en direction de l'extrémité distale de la lumière de chargement (22), et
la partie d'insertion (27a) est configurée de telle façon qu'une forme de flexion de la partie de flexion (27c) est corrigée lorsque la partie d'insertion (27a) est insérée dans l'embout femelle de gaine (43), et dans l'état monté, la partie de contact distale (27b) et la surface intérieure de la partie conique (43d) viennent en contact l'une avec l'autre, et l'axe de la lumière de chargement (22) et l'axe de la lumière de gaine (42) du cathéter d'administration (40) sont alignés.

7. Système d'administration (200) selon l'une quelconque des revendications 1 à 6, dans lequel
la partie d'insertion (27a) de la partie de connexion distale (27) est insérée dans la partie de communication au diamètre élargi (43c) de telle façon que l'extrémité distale de la lumière de chargement (22) est adjacente à la partie de communication distale (43b) dans l'état monté.

8. Système d'administration (200) selon l'une quelconque des revendications 1 à 7, dans lequel
le corps de cathéter (21) du cathéter de chargement de matériel d'embolisation (20) a une rigidité supérieure à celle de la gaine (41) du cathéter d'administration (40).
